# NEUE EUROPÄISCHE PATENTSCHRIFT

(11) **EP 2 467 273 B2**
(45) Veröffentlichungstag und Bekanntmachung des Hinweises auf die Entscheidung über den Einspruch: **13.03.2019**
(45) Hinweis auf die Patenterteilung: 20.04.2016
(21) Anmeldenummer: 10742473.1
(22) Anmeldetag: 06.08.2010
(51) Int. Cl.: B60H 1/00, B60H 3/00

(54) **VERFAHREN ZUR ANSTEUERUNG EINER IONISIERUNGSVORRICHTUNG**
METHOD OF CONTROLLING AN IONISATION DEVICE
PROCÉDÉ DE CONTROLE D'UN APPAREIL D'IONISATION

(30) Priorität: 21.08.2009 DE 102009038296
(43) Veröffentlichungstag der Anmeldung: 27.06.2012
(73) Patentinhaber: MAHLE Behr GmbH & Co. KG, 70469 Stuttgart (DE)
(72) Erfinder: RAIS, Thomas, 71672 Marbach/Neckar (DE); PITZ, Eric, 70199 Stuttgart (DE)
(74) Vertreter: Grauel, Andreas
(86) Internationale Anmeldenummer: PCT/EP2010/061472
(87) Internationale Veröffentlichungsnummer: WO 2011/020711

(56) Entgegenhaltungen:
- EP-A1- 1 323 589
- WO-A1-2006/134681
- DE-B- 1 261 295
- JP-A- 2002 277 010
- JP-A- 2004 079 471
- JP-A- 2004 182 123
- US-A- 3 887 846
- US-A- 4 542 434
- US-A- 5 153 811
- US-A1- 2007 109 711

## Beschreibung

Die Erfindung betrifft ein Verfahren zur Ansteuerung einer Ionisierungsvorrichtung gemäß dem Oberbegriff von Anspruch 1. Auch betrifft die Erfindung eine Klimaanlage nach dem Oberbegriff von Anspruch 6.

Um die im Zusammenhang mit der Belüftung von Kraftfahrzeugen über die Jahre hinweg gestiegenen Komfortanforderungen der Fahrzeugkunden zu befriedigen, hat die Funktionsvielfalt und die Komplexität von Kraftfahrzeugklimaanlagen entsprechend zugenommen.

So haben sich Klimaanlage zwischenzeitlich in sämtlichen Fahrzeugklassen durchgesetzt. Mit derartigen Fahrzeugklimaanlagen kann die dem Fahrzeuginnenraum zuzuführende Frischluft erwärmt werden. Heutzutage ist jedoch meist auch eine Kühlfunktion vorgesehen, mit der die dem Fahrzeuginnenraum zuzuführende Frischluft auch abgekühlt werden kann.

Weitere Funktionen, die zwischenzeitlich in zunehmendem Umfang bei der Belüftung von Kraftfahrzeugen realisiert werden, sind beispielsweise Filter, die die dem Fahrzeuginnenraum zuzuführende Frischluft reinigen und/oder zur Entfernung von unerwünschten Gerüchen (insbesondere Außenluftgerüchen) eingesetzt werden.

Seit einiger Zeit werden in Kraftfahrzeugen auch im Rahmen der Erstausrüstung Ionisierungsgeräte verbaut. Die Ionisierungsgeräte sollen einerseits für eine Luftauffrischung sorgen, also für ein Empfinden, dass eine "frische Luft" vorliegt. Andererseits werden Ionisierungsgeräte dazu verwendet, um Krankheitserreger (insbesondere Bakterien und/oder Viren), welche sich gegebenenfalls in der dem Fahrzeuginnenraum zuzuführenden Frischluft und/oder Umluft befinden, abzutöten. Um Krankheitserreger abzutöten wird meist eine Kombination aus einer positiv geladenen Elektrode sowie einer negativ geladenen Elektrode verwendet. Als wirksames Ion dient dabei häufig HO₂⁻. Um HO₂⁻ zu erzeugen wird aus Wasser (H₂O), welches in Form von Luftfeuchtigkeit vorliegt, mit Hilfe einer positiv geladenen Elektrode H⁺ erzeugt, welches anschließend an der negativ geladenen Elektrode zu H reduziert wird. H kombiniert anschließend mit O₂⁻ zum wirksamen HO₂⁻.

Zur Lufterfrischung werden dagegen meist negativ geladene (einfache) Hochspannungselektroden verwendet, welche zum Beispiel in der zuzuführenden Luft enthaltene Sauerstoffmoleküle (O₂) zu negativ geladenen Sauerstoffionen reduzieren (O₂⁻).

Damit die Ionisierungsgeräte ihre Wirksamkeit entfalten können, ist es nicht nur erforderlich, dass diese Ionen erzeugen, sondern dass die von den Ionisierungsgeräten erzeugten Ionen auch über eine längere Zeitdauer hinweg vorhanden bleiben und vorteilhafterweise in den zu belüftenden Innenraum freigesetzt werden. Dies ist insbesondere im Kraftfahrzeugbereich ein Problem, wo die aufbereitete Luft über eine größere Anzahl von Luftleitelementen, Luftleitklappen und Auslassdüsen gelenkt wird, bevor die aufbereitete Luft in den Kraftfahrzeuginnenraum abgegeben wird. Versuche haben ergeben, dass sich eine anfänglich ausreichend hohe Ionenkonzentration innerhalb von wenigen Minuten um mehr als den Faktor 10 verringern kann. Dies verringert die Wirksamkeit der Luftionisierung und macht entsprechende Gegenmaßnahmen erforderlich.

In JP 20063492989 A wird beispielsweise vorgeschlagen, einen flexiblen Luftleitschlauch einer Klimaanlage, die eine Ionisierungsvorrichtung aufweist, mit einer durchgängigen, elektrisch leitfähigen Beschichtung zu versehen. Die elektrisch leitfähige Beschichtung wird geerdet. Im vorgeschlagenen Aufbau soll verhindert werden, dass die durch den Schlauch hindurch strömenden Ionen elektrisch neutralisiert werden, und damit verloren gehen.

In JP 2004-79471 A wird ein Verfahren beschrieben, mit dem der Verlust bereits erzeugter Anionen in einem, der Ionisierungsvorrichtung nachfolgenden Luftleitkanal, verhindert werden soll. Dazu wird vorgeschlagen, dass die Ionisierungsvorrichtung nach einem durchgängigen Betrieb über eine Zeitdauer von 10 bis 40 Minuten hinweg für etwa 3 bis 7 Minuten ausgeschaltet wird.

Im WO 2009/045430 A1 werden unterschiedliche organische Polymerzusammensetzungen, die mit einem metallischen Überzug versehen werden, für die Verwendung als Luftführungskanäle in Kraftfahrzeugen vorgeschlagen.

Aus dem Dokument JP 2004 82123 sind ein Verfahren nach dem Oberbegriff des Anspruchs 1 und eine Klimaanlage für ein Kraftfahrzeug mit einer Ionenerzeugungsvorrichtung bekannt, bei der in einem Säuberungsmodus negative und positive Ionen und in einem Refresh-Modus nur negative Ionen erzeugt werden können.

Aus dem Dokument WO 2006/134681 A1 ist eine Verbindungsstruktur eines Kanals zum Transport von Gas bekannt. Es wird ein Ionenerzeuger vorgeschlagen, der elektrisch mit einer Metall-enthaltenen Öffnung verbunden ist, und wobei das innere Kernmaterial seines flexiblen Kanals geerdet ist.

Das Dokument JP 2002 277010 offenbart eine Klimaanlage mit einem Lüfter, um atmosphärische Luft einzusaugen, wobei die Luft an einen Luftführendem Kanal geliefert wird, einem Filter zum Entfernen von Staub in der Luft und einem negativen Ionenerzeuger, der eine lineare Hochspannungselektrode und ein eine Metall-Erdungselektrode aufweist. Der Ionenerzeuger kann negative Ionen bereitstellen.

Das Dokument EP 1 323 589 A1 offenbart eine Heizungs- und Klimaanlage, insbesondere für ein Kraftfahrzeug, mit einem Lüftungsgehäuse, in und 7 oder an dem Komponenten der Anlage angeordnet sind, wobei die Komponenten Gebläse, gegebenenfalls Filter, wenigstens einen Wärmetauscher, Luftstromsteuerelemente, Aktoren und gegebenenfalls Sensoren umfassen. Ferner ist eine Steuerelektronik vorgesehen, in die von einer Bedieneinheit Steuersignale eingebbar sind und die Steuerelektronik über elektrische Leitungen mit wenigstens einigen der Komponenten der Anlage verbunden ist, wobei zumindest ein Teil der elektrischen Leitungen durch eine oder mehrere Folienleiterbahnen gebildet wird.

Obwohl die bereits vorgeschlagenen Maßnahmen grundsätzlich geeignet sind, das beschriebene Problem des Verlusts von bereits erzeugten Ionen zu verringern, weisen die bereits vorgeschlagenen Verfahren und Vorrichtungen jedoch nach wie vor unterschiedlichste Probleme auf, wie beispielsweise hohe Kosten, nicht ausreichende Wirksamkeit und übermäßige elektrische Neutralisierung bereits erzeugter Ionen.

Es ist daher die Aufgabe der Erfindung, ein Verfahren zur Ansteuerung einer Ionisierungsvorrichtung zur Ionisierung von Luft zur Belüftung von Kraftfahrzeuginnenräumen vorzuschlagen, welches die bei im Stand der Technik bekannten Verfahren verbessert. Eine weitere Aufgabe der Erfindung besteht darin, eine Klimaanlage mit einer Ionisierungsvorrichtung zur Ionisierung von Luft zur Belüftung von Kraftfahrzeuginnenräumen vorzuschlagen, welche die im Stand der Technik bekannten Ionisierungsvorrichtungen verbessert.

Die Aufgabe zu dem Verfahren wird mit den Merkmalen von Anspruch 1 gelöst. Die Aufgabe zur Klimaanlage wird mit den Merkmalen von Anspruch 6 gelöst.

Es wird daher vorgeschlagen, ein Verfahren zur Ansteuerung einer Ionisierungsvorrichtung zur Ionisierung von Luft zur Belüftung von Kraftfahrzeuginnenräumen, wobei die Ionisierungsvorrichtung zeitweise in einem normalen Betriebsmodus Ionen eines ersten Ionentyps freisetzt, wobei die Ionisierungsvorrichtung zeitweise in zumindest einem Regenerationsmodus betrieben wird, in dem Ionen mit einem zweiten, vom ersten Ionentyp unterschiedlichen Ionentyp freigesetzt werden, wobei die Ionisierungsvorrichtung in aufeinanderfolgenden Zyklen betrieben wird, in denen die Ionisierungsvorrichtung jeweils für einen gewissen Zeitraum zunächst im normalen Betriebsmodus, dann im Regenerationsmodus betrieben wird.

Beispielsweise kann die Ionisierungsvorrichtung eine geeignet ausgebildete Elektrode aufweisen, an welche eine negative Hochspannung angelegt wird, Die Elektrode setzt dann negativ geladene Ionen frei, also sogenannte Anionen. Negative Ionen enthaltende Luft wird in aller Regel als "frische Luft" wahrgenommen. Dementsprechend wird üblicherweise auch von einer sogenannten "Relax"-Betriebsart (Relax für Entspannen) gesprochen. Ebenso ist es auch möglich, dass bei der Ionisierungsvorrichtung eine geeignet geformte Elektrode vorgesehen wird, an welcher eine positive Hochspannung angelegt wird. Die derart positiv geladene Hochspannungselektrode emittiert dementsprechend positiv geladene Ionen, sogenannte Kationen. Die erzeugten Kationen (zum Beispiel H⁺-Ionen) können mit anderen Molekülen und/oder Ionen kombinieren (zum Beispiel mit O₂⁻-Ionen zu HO₂⁻-Ionen), die ihrerseits reinigend (z. Bsp. bakteriozid) wirken können. Deshalb wird hier üblicherweise von einem "Clean"-Betriebsmodus (Clean für sauber) gesprochen. Selbstverständlich ist es möglich, dass auch mehrere Elektroden (sowohl mit gleicher Hochspannungsversorgung, als auch mit unterschiedlicher Hochspannungsversorgung) vorgesehen werden. Unabhängig von der konkreten Ionenart kommt es insbesondere bei der alleinigen bzw. überwiegenden Freisetzung von Anionen bzw. Kationen (welche gegebenenfalls auch nur als "Zwischenstufe" temporär vorliegen können) und/oder von bestimmten Ionentypen (wie beispielsweise O₂⁻-Ionen und/oder HO₂⁻-Ionen) in aller Regel zu einer elektrostatischen Aufladung von Bauteilen, die in Luftströmungsrichtung gesehen der Ionisierungsvorrichtung nachgeschaltet sind. Hierbei kann es sich beispielsweise um Luftführungselemente, Luftleitklappen, Luftausströmerdüsen und dergleichen handeln. Aufgrund der im Luftstrom enthaltenen Ionen werden die entsprechenden Bauteiloberflächen elektrostatisch geladen. Dadurch entstehen elektrische Felder, welche wiederum einen Einfluss auf die Luftströmung, insbesondere auf die in der Luftströmung enthaltenen Ionen, haben. Somit entsteht ein hochkomplexes, auch numerisch kaum beschreibbares dynamisches Gebilde. Die unterschiedlichen dynamischen Ladungsverteilungen bewirken jedoch in aller Regel eine "Behinderung" der im Luftstrom mitgeführten Ionen (meist dadurch, dass die Ionen abgelenkt werden und anschließend durch einen Wandkontakt vernichtet werden), Experimente haben dabei ergeben, dass sich bereits nach wenigen Minuten eine drastische Verringerung der schlussendlich in einen Kraftfahrzeuginnenraum freigesetzten Ionenkonzentration ergeben kann. Die Zeitspanne, nach der es zu einer signifikanten Einbuße an freigesetzten Luftionen (bis zu einem Faktor 10 und mehr) kommen kann, ist dabei überraschenderweise deutlich kürzer als bislang angenommen. Versuche haben ergeben, dass die Reduktion der freigegebenen Ionen um einen Faktor 10 bereits nach 1 bis 5 Minuten erreicht wird. Von daher ist es auch bei typischen Betriebszyklen bei einem Kraftfahrzeug (beispielsweise bei einem Automobil) erforderlich, entsprechende Gegenmaßnahmen vorzusehen, die der beschriebenen Verringerung der Anzahl der in den Kraftfahrzeuginnenraum freigesetzten Ionen entgegen wirkt. Vorliegend wird vorgeschlagen, dass hierfür geeignete Regenerationsphasen vorgesehen werden. Während der Regenerationsphasen kann dabei durchaus bewusst in Kauf genommen werden, dass sich über einen gewissen - möglichst kurzen - Zeitraum hinweg das (eigentlich) gewünschte Konzentrationsgemisch und/oder die Konzentration der in den Kraftfahrzeuginnenraum freigesetzten Ionen ändert. Es ist sogar denkbar, dass kurzzeitig die Anzahl der freigesetzten Ionen (zumindest bei einigen Ionentypen) auf 0 zurückgefahren wird. Dennoch ist es trotz des Einfügens derartiger Regenerationsphasen möglich, die im zeitlichen Mittel freigesetzte Menge an Luftionen dank der Regenerationsphasen zu erhöhen bzw. das Konzentrationsverhältnis der in den Kraftfahrzeuginnenraum freigesetzten Ionen dem gewünschten Zielwert besonders nahe kommen zu lassen. Insbesondere ist dies dadurch möglich, dass während der "aktiven" Phasen, die zwischen den Regenerationsphasen liegen, beispielsweise eine vergleichsweise hohe Konzentration an Ionen in den Kraftfahrzeuginnenraum abgegeben werden kann. In der Regel wirkt die Regenerationsphase dabei weniger auf das eigentliche Ionisierungsmittel, als vielmehr auf die dem Ionisierungsmittel im Luftstrom nachgeschalteten Bauteile (beispielsweise Luftführungskanäle und/oder Luftauslassdüsen).

Gemäß der Erfindung wird das Verfahren derart durchgeführt, dass bei zumindest einem Regenerationsmodus zumindest zeitweise Ionen mit einem zweiten, vom ersten Ionentyp unterschiedlichen Innentyp freigesetzt werden-Unter einem Ionentyp kann ein unterschiedliches Vorzeichen der Ladung zu verstehen sein. Wenn also beispielsweise die Ionen des ersten Ionentyps Anionen sind, so kann es sich dementsprechend bei den Ionen vom zweiten Ionentyp um Kationen handeln (die gegebenenfalls auch nur In Form einer "Zwischenstufe" temporär vorliegen können). Zusätzlich oder alternativ können die Ionen jedoch auch eine unterschiedliche Ladung (beispielsweise einfache, zweifache, dreifache usw. Elementarladung) aufweisen. Insbesondere kann es sich zusätzlich oder alternativ auch um unterschiedliche Moleküle handeln, die ionisiert werden beziehungsweise ionisiert wurden (wie beispielsweise die bereits vorab beschriebenen Ionen O₂⁻ und HO₂⁻), Insbesondere dann, wenn Ionen mit entgegengesetzter Ladung während des Regenerationsmodus freigesetzt werden, können die gegebenenfalls bereits elektrostatisch geladenen Bereiche der dem eigentlichen Ionisierungsmittel nachgeschalteten Bauteile durch die Platzierung entgegengesetzter Ladungsträger entladen werden. Auch ist es möglich, quasi eine entgegengesetzte Vorab-Ladungsverteilung zu erzeugen, so dass in einer ersten, der Regenerationsphase nachgeschalteten Betriebsphase der Ionisierungsvorrichtung zunächst die dem eigentlichen Ionisierungsmittel nachgeschalteten Bauteile entladen werden, und erst anschließend durch die Ionen des ersten Ionentyps neu aufgeladen werden. Insbesondere dann, wenn eine Regeneration mit Hilfe von Ionen eines zweiten Ionentyps erfolgt, kann die Dauer der Regenerationsphase typischerweise in einem Bereich zwischen 5 und 30 Sekunden liegen, während die "normale" Betriebsdauer in einem Intervall zwischen 30 Sekunden und 3 Minuten liegt.

Möglich ist es, dass bei den Verfahren die Ionen vom zweiten Ionentyp zumindest zeitweise zusätzlich und/oder zumindest zeitweise alternativ zu den Ionen des ersten Ionentyps freigesetzt werden. Es ist also möglich, dass (zumindest zeitweise) während der Regenerationsphase vorwiegend oder (im Wesentlichen) ausschließlich Ionen vom zweiten Ionentyp freigesetzt werden. In diesem Fall kann die Dauer der Regenerationsphase typischerweise besonders kurz gehalten werden. Ebenso ist es jedoch auch möglich, dass die Ionen vom zweiten Ionentyp freigesetzt werden, während die Erzeugung von Ionen vom ersten Ionentyp im Wesentlichen kontinuierlich fortgeführt wird. Hierdurch kann eine höhere Konstanz an Freisetzung von "frischer Luft" in den Fahrzeuginnenraum hinein erreicht werden. Darüber hinaus kann in der Regel die Ansteuerung der entsprechenden Elektrode einfacher gestaltet werden.

Besonders vorteilhaft kann es sein, wenn bei zumindest einem weiteren Regenerationsmodus zeitweise keine Ionen, insbesondere zumindest zeitweise keine Ionen vom ersten Ionentyp freigesetzt werden. Diese Fortbildung des Verfahrens kann beispielsweise durch ein vollständiges Ausschalten der Ionisierungsvorrichtung realisiert werden. Auch hat es sich gezeigt, dass bereits nach relativ kurzen Zeitdauern eine Regeneration der Gesamtanordnung erfolgen kann. Typischerweise reichen Ausschaltzeiten in einem Bereich zwischen 5 und 30 Sekunden aus (bei Einschaltzeiten im normalen Betriebsmodus von typischerweise 30 Sekunden bis 3 Minuten). Diese Lösung ist insbesondere deshalb von Vorteil, weil sie konstruktiv besonders einfach realisiert werden kann. Insbesondere kann dieses Verfahren für Ionisierungsvorrichtungen verwendet werden, die lediglich eine einzelne Hochspannungsquelle und/oder eine einzelne Hochspannungselektrode aufweisen. Eine besonders schnelle Regeneration kann dabei insbesondere dann realisiert werden, wenn relativ feuchte Luft vorliegt, da diese eine schnelle Entladung elektrostatischer Ansammlungen begünstigt.

Vorzugsweise wird das Verfahren derart ausgeführt, dass zumindest zeitweise und/oder zumindest bereichsweise eine lokale Feldkompensation von elektrischen Feldern mit Hilfe von Spiegelladungen erfolgt, insbesondere unter Verwendung von passiv wirkenden konstruktiven Maßnahmen. Die Erfinder haben zu ihrer eigenen Überraschung herausgefunden, dass nicht etwa besonders gut leitende, flächig aufgetragene Materialien und/oder besonders gut isolierende Oberflächen eine besonders hohe Konzentration von freigesetzten Ionen bewirkt. Vielmehr hat sich gezeigt, dass ein besonders hoher Ionenaustrag dann erfolgen kann, wenn die der Ionisierungsvorrichtung nachgeschalteten Bauteile über größere Entfernungen hinweg eine relativ niedrige elektrische Leitfähigkeit aufweisen und/oder in einem kleinräumigen Bereich eine relativ hohe elektrischen Leitfähigkeit aufweisen. Dadurch kann eine nur lokal wirkende elektrische Feldkompensation realisiert werden, wohingegen im großräumigen Bereich keine elektrische Feldkompensation (bzw. eine nur geringe bzw. langsame elektrische Feldkompensation) erfolgen kann. Die Größenordnung der lokalen Strukturen, in denen eine signifikante Feldkompensation erfolgen kann, liegt dabei typischerweise in einem Bereich von wenigen Zentimetern (bzw. Quadratzentimetem), beispielsweise mit typischen Längen und/oder Breiten von 0,5 cm, 1 cm, 1,5 cm, 2 cm, 3 cm, 4 cm oder 5 cm. Vorteilhaft ist es, wenn die lokale Feldkompensation durch passiv wirkende konstruktive Maßnahmen erfolgt. Derartige passiv wirkende konstruktive Maßnahmen sind in aller Regel besonders einfach und kostengünstig realisierbar. Beispielhafte Ausgestaltungen derartiger passiv wirkender konstruktiver Maßnahmen sind insbesondere die im Folgenden noch eingehender beschriebenen Ausbildungen in Form einer Mehrzahl von elektrisch leitfähigen Materialabschnitten, einem oder mehreren durchgängigen, elektrisch geringfügig leitenden Bauteilen, flächigen bzw. folienartigen Ausbildungen und/oder selbstklebende Ausführungen, die im Zusammenhang mit der im Folgenden eingehend beschriebenen Ionisierungsvorrichtung beschrieben werden.

Vorzugsweise wird das Verfahren derart durchgeführt, dass der Regenerationsmodus zeitgesteuert erfolgt und typischerweise länger als 2 Sekunden, 5 Sekunden, 10 Sekunden, 20 Sekunden, 30 Sekunden, 45 Sekunden und/oder 1 Minute und/oder typischerweise kürzer als 20 Sekunden, 30 Sekunden, 45 Sekunden, 1 Minute, 2 Minuten, 3 Minuten, 4 Minuten und/oder 5 Minuten andauert. Eine zeitgesteuerte Realisierung des Regenerationsmodus ist üblicherweise besonders einfach. Insbesondere werden in aller Regel keine zusätzlichen Bauteile wie insbesondere Sensoren oder dergleichen benötigt. Die gennannten Zeitdauern haben sich in ersten Versuchen als besonders vorteilhaft erwiesen.

Weiterhin wird gemäß Anspruch 6 eine Klimaanlage mit einer Ionisierungsvorrichtung zur Ionisierung von Luft zur Belüftung von Kraftfahrzeuginnenräumen vorgeschlagen, die Ionisierungsmittel zur zeitweisen Freisetzung von Ionen unterschiedlichen Ionentyps aufweist, wobei die lonisierungsvorrichtung derart ausgebildet und eingerichtet ist, dass sie zumindest zeitweise nach einem erfindungsgemäßen Verfahren betreibbar ist.

Eine derartige Ionisierungsvorrichtung weist einen gegenüber bekannten Ionisierungsvorrichtungen besonders hohen zeitgemittelten Ausstoß an Ionen auf, und weist dabei gleichzeitig einen relativ einfachen konstruktiven Aufbau der Anordnung auf.

Vorteilhaft ist es, wenn bei der Ionisierungsvorrichtung zumindest eine Steuervorrichtung zur Ansteuerung der Ionisierungsvorrichtung, insbesondere eine Steuervorrichtung zur Durchführung des Regenerationsmodus vorgesehen ist. Als derartige Steuervorrichtung kann beispielsweise ein Einplatinencomputer oder dergleichen dienen. Insbesondere ist es möglich, dass die Funktionalität von einer bei Kraftfahrzeugen typischerweise ohnehin vorhandenen elektronischen Steuerung (beispielsweise einer elektronischen Steuerung für eine Kraftfahrzeugklimaanlage) mit übernommen wird. Da die Rechenlast für die Steuervorrichtung typischerweise relativ klein ist, stellt diese zusätzliche Arbeitslast in aller Regel kein Problem dar.

Weiterhin wird vorgeschlagen, die Ionisierungsvorrichtung mit zumindest einem zweiten Ionisierungsmittel zu versehen, insbesondere mit einem Ionisierungsmittel zur zumindest zeitweisen Freisetzung von Ionen eines zweiten, vom ersten Ionentyp unterschiedlichen Ionentyps. Mit einer derartigen Anordnung kann der entsprechende, bereits vorab beschriebene Regenerationsmodus besonders vorteilhaft ausgeführt werden.

Vorteilhaft ist es weiterhin, wenn die Ionisierungsvorrichtung zumindest eine Luftführungsvorrichtung, insbesondere zumindest einen Luftführungskanal und/oder zumindest eine Luftauslassdüse aufweist, welche zumindest bereichsweise ein lokal wirkendes Feldkompensabensmittel aufweiset Mit einem derartigen Aufbau ist es möglich, einen besonders hohen Anteil der von der Ionisierungsvorrichtung erzeugten Ionen in einen Kraftfahrzeuginnenraum auszugeben. Eine entsprechend ausgebildete Ionisierungsvorrichtung erweist sich somit als besonders effektiv. Das oder die lokalen Feldkompensationsmittel können dabei in beliebiger Weise auf der Außenseite, der Innenseite und/oder in einer Zwischenlage der Luftführungsvorrichtung (wie beispielsweise eines Luftführungskanals und/oder einer Luftauslassdüse) ausgebildet werden.

Möglich ist es, dass bei der Ionisierungsvorrichtung zumindest ein lokal wirkendes Feldkompensationamittel zumindest bereichsweise als eine Mehrzahl von elektrisch leitfähigen Malerialabschnitten ausgebildet ist. Bei den Materialabschnitten kann es sich beispielsweise um Metallplättchen, Metallklötzchen oder ähnliche Bauteile handeln. Ein derartiges lokal wirkendes Feldkompensationsmittel weist eine nur geringe Tendenz zur Vernichtung von Ionen, bei gleichzeitig retativ einfachem und kostengünstigem Aufbau auf. Im Übrigen soll es auch möglich sein für die vorgeschlagene. Ausbildung auch ohne den Begriff "lokal wirkendes Feldkompesnsationsmittel" Schutz erlangen zu können.

Zusätzlich oder alternativ ist es auch möglich, dass zumindest ein lokal wirkendes Feldkompensationsmittel zumindest bereichsweise als durchgängiges, elektrisch geringfügig leitendes Bauteil ausgebildet ist Hier können beispielsweise sogenannte ESD-Folien oder dergleichen Bauteile verwendet werden (ESD für Electro Static Device). Auch hier hat sich gezeigt, dass derartige Bauteile in besonderem Maße als lokales Feldkompensationsmittel geeignet sind und dementsprechend ein besonders hoher Anteil der erzeugtten Ionen von den entsprechenden Bauteilen "durchgelassen" wird. Dennoch kann der Aufbau der entsprechenden Ionisierungsvorrichtung nach wie vor relativ einfach und kostengünstig sein. Auch in diesem Zusammenhang soll es möglich sein für die vorgeschlagene Ausbildung auch ohne den Begriff "lokal wirkendes Feldkompensationsmittel" Schutz erlangen zu können.

Weiterhin hat es sich als vorteilhaft erwiesen, wenn bei der Ionisrerungsvorrichtung zumindest ein lokal wirkendes Feldkompensationsmittel, insbesondere zumindest ein elektrisch leitfähiger Materialabschnitt und/oder zumindest ein elektrische geringfügig leitendes Bauteil flächig, bevorzugt folienartig ausgebildet ist. Erste Versuche haben ergeben, dass es bei den lokal wirkenden Feldkompensationsmitteln in aller Regel nicht (oder nur in geringfügigem Ausmaß) auf deren Tiefe (insbesondere deren Bauteilabmessungen in einer sankrerht zur Luftströmungsrichtung stehenden Richtung) ankommt. Mit einem flächigen bzw. folienartigen Aufbau kann daher eine hochwirksame Vorrichtung bei geringem Materialverbrauch (und damit niedrigem Gewicht und/oder geringen Kosten) ausgebildet werden. Typische Dicken von flächig ausgebildeten Bauteilen und/oder Folien liegen im Bereich von 0,1 mm.

Weiterhin kann es sich als vorteilhaft erweisen, wenn bei der Ionisierungsvorrichtung zumindest ein lokal wirkendes Feldkompensationsmittel, insbesondere zumindest ein elektrisch leitfähiger Materialabschnitt und/oder zumindest ein elektrisch geringfügig leitendes Bauteil selbstklebend ausgeführt ist. Bei einer derartigen Bauausführung kann eine besonders einfache Montage der lokal wirkenden Feldkompensationsmittel an den übrigen Baugruppen realisiert werden. Darüber hinaus ist darauf hinzuweisen, dass beispielsweise Metallfolien und/oder ESD-Folien oftmals ohnehin in selbstklebender Form handelsüblich erhältlich sind. Dadurch kann eine nochmalige Kostenreduktion erzielt werden.

Die Klimaanlage weist dann die bereits im Zusammenhang mit der vorab beschriebenen Ionisierungsvorrichtung beschriebenen Eigenschaften und Vorteile in analoger Weise auf. Bei dem Kraftfahrzeug, für das die Kraftfahrzeugklimaanlage bestimmt ist, kann es sich in beliebiger Weise um ein Wasserfahrzeug, ein Luftfahrzeug und/oder ein Landfahrzeug (schienengebunden/nicht schienengebunden) handeln.

Im Folgenden wird die Erfindung anhand vorteilhafter Ausführungsbeispiele und unter Bezugnahme auf die beigefügte Zeichnung näher erläutert, Es zeigen:
- Fig. 1:: eine Kraftfahrzeugklimaanlage mit einer Ionisieungsvorrichtung im schematischen Querschnitt;
- Fig. 2:: der zeitliche Verlauf der von einer Kraftfahrzaugktimaantage in den Kraftfahrzeuginnenraum ausgegebenen Ionenkonzentration in einem Dauerbetriebsverfahren;
- Fig. 3:: der zeitliche Verlauf der von einer Kraftfahrzeugklimaanlage in den Kraftfahrzeuginnenraum ausgegebenen Ionenkonzentration unter Verwendung eines ersten Typs von Regenerationsverfahren;
- Fig. 4:: der zeitliche Verlauf der von einer Kraftfahrzeugklimaanlage in den Kraftfahrzeuginnenraum ausgegebenen Ionenkonzentration unter Verwendung eines zusätzlichen Typs von Regenerationsvorfahren;
- Fig, 5:: ein erstes Ausführungsbeispiel für einen Luftleitkanal mit lokal wirkendem Foldkompensationsmittel in schematiseher, perspektivischer Ansicht;
- Fig. 6:: ein zweites Ausführungsbeispiel für einen Luftührungskanal mit lokal wirkendem Feldkompensationsmittel in schematischer, perspektivischer Ansicht

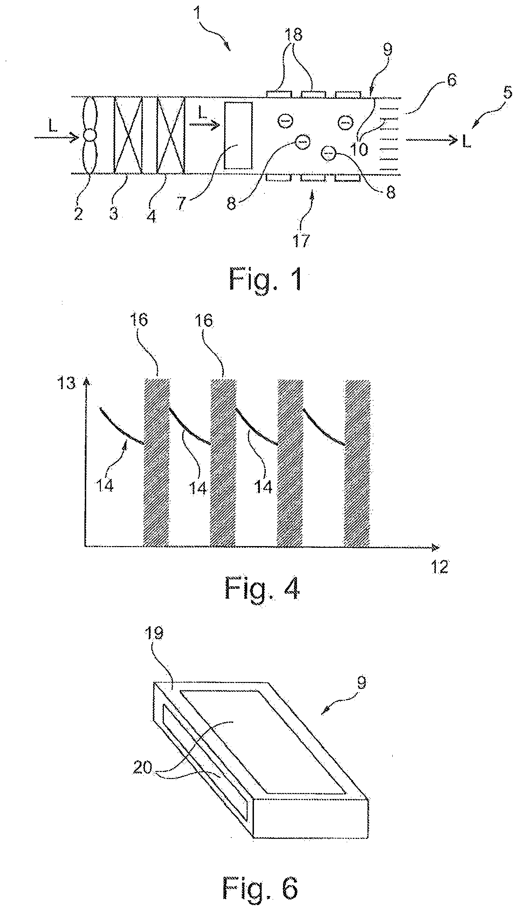

In Fig. 1 ist eine Kraftfahrzeugklimaanlage in einer schematischen Querschnittsansicht dargestellt. Die Kraftfahrzeugklimaanlage 1 ist dabei in einer stark vereinfachenden Ansicht dargestellt, die sich im Wesentlichen auf die Bereiche konzentriert, die im Zusammenhang mit der Lüftaufbereitung durch Ionisierung der von der Kraftfahrzeugklimaanlage 1 aufzuarbeitenden Luftströmung L stehen.

Die von der Kraftfahrzeugklimaanlage 1 mit Hilfe eines Gebläses 2 angesaugte Luft L wird zunächst durch einen Filter 3 geleitet, in dem Pollen, Schmutzpartikel und dergleichen, die in der Umgebungsluft enthalten sein können, ausgefiltert werden. Anschließend wird die Luftströmung L durch einen Heizkörper 4 geleitet und entsprechend temperiert (selbstverständlich kann die Temperierung auch durch eine Mischung von Warm- und Kaltluft erfolgen; Darüber hinaus kann die Luft auch mit Hilfe eines Verdampfers gekühlt werden usw.). Die bereits weitgehend aufbereitete Luftströmung L wird, bevor sie über eine Luftausströmerdüse 6 in einen Kräfifahrzeuginnenraum 5 freigegeben wird, zunächst durch ein Ionisierungsmodul 7 geleitet. In diesem Ionisierungsmodul 7 werden beispielsweise Anionen 8 erzeugt, die von den Kraftfahrzeuginsassen als "frische Luft" wahrgenommen werden. Die vom Ionisierungsmodul 7 erzeugten Anionen müssen dabei zunächst über entsprechend geformte Luftleftkanäle 9 hindurch geführt werden sowie durch die Luftausströmerdüse 6 hindurch strömen. Dabei treffen einige der vom Ionisierungsmodul 7 erzeugten Anionen 8 auf die Wände des Luftleitkanals 9 bzw. der Luftausströmerdüse 6 auf und geben ihre Ladung an den entsprechenden Wandbereich 10 ab. Mit der Zeit kommt es dadurch zu einer elektrostatischen Aufladung bestimmter Wandbereiche 10 von Luftleitelement 9 und Luftausströmerdüse 6, Die elektrostatische Aufladung der entsprechenden Wandbereiche 10 ist jedoch nicht gleichmäßig, sodass zwischen unterschiedlich stark geladenen Wandbereichen 10 elektrische Felder auftraten. Die elektrischen Felder beeinflussen wiederum die Luftdurchströmung L (speziell die in der Luftdurchströmung L enthaltenen Ionen), insbesondere die Bewegungsbahn der Anionen 8, sodass sich das System überaus dynamisch und nur schwer vorhersagbar verhält.

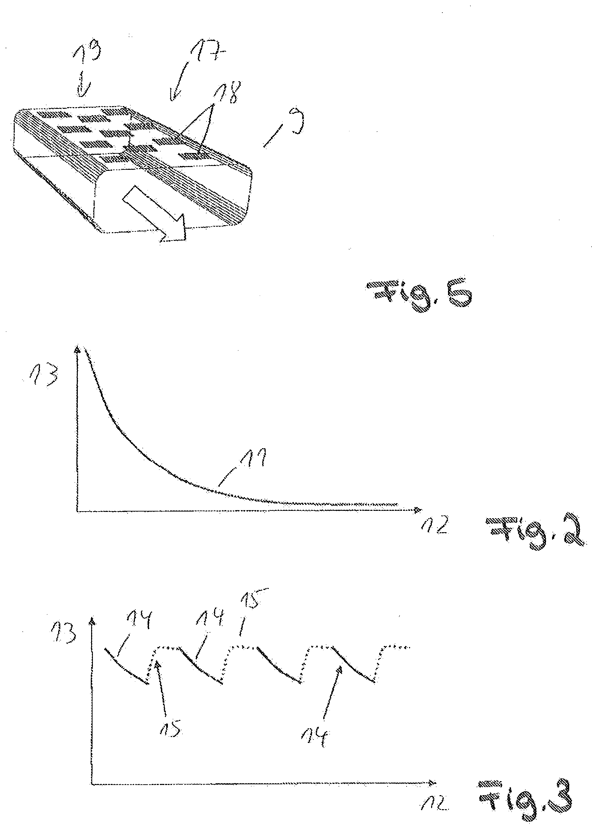

In aller Regel bewirkt die elektrostatische Aufladung der Wandbereiche 10 von Luftleitkanal 9 und Luftausströmerdüsen 6 jedoch eine starke Reduktion der in den Kraftfahrzeuginnenraum 5 schlussendlich austretende Anionen 8. Dementsprechend müsste das Ionisierungsmodul 7 eine entsprechend größere Anzahl an Anionen 8 erzeugen. Dafür müsste das Ionisierungsmodul 7 entsprechend größer ausgelegt Werden (wodurch es teurer und schwerer würde), und entsprechend mehr elektrische Energie müsste für das Ionisierungsmodul 7 bereit gestellt werden. Die Abnahme der Konzentration der in den Kraftfahrzeuginnenraum 5 freigegebenen Anionen 8 ist in Fig. 2 schematisch als Fünktionsgraf 11 dargestellt. In Fig. 2 ist entlang der Abszisse 12 die Zeit t dargestellt, wohingegen entlang der Ordinate 13 die Ionenkonzentration, die in den Kraftfahrzeuginnenraum 5 freigegeben wird, aufgetragen ist Deutlich erkennt man die starke Abnahme der Ionenkonzentration. Typischerweise erreicht nach einer Zeitdauer von 1 bis 5 Minuten nur noch jedes zehnte erzeugte Anion 8 den Kraftfahrzeuginnenraum 5. Der Effekt ist also überaus signifikant,

Um die im zeitlichen Mittel in den Kraftfahrzeuginnenraum 5 freigegebene Konzentration an Anionen 8 zu erhöhen, wird vorgeschlagen, das Ionisierungsmadul 7 nur über einen gewissen Zeitraum (typischerweise 1 bis 3 Minuten) hinweg in einem normalen Betriebsmodus 14 zu betreiben (vergleiche mit Fig. 3). Nach Ablauf einer gewissen Zeitspanne wird die Kraftfahrzeugklimaanlage 1 in einen Regenerationsmodus 15 geschaltet, in dem nicht nur Anionen 8 (wie in Fig. 1 dargestellt) erzeugt werden, sondern in dem zusätzlich auch Kationen von dem Ionisierungsmodul 7 (was hierfür entsprechend ausgestaltet sein muss) erzeugt werden. Die vom Ionisierungsmodul 7 erzeugten Kationen werden im vorliegend dargestellten Ausführungsbeispiel nur "temporär" erzeugt und dienen lediglich zur Erzeugung eines zweiten Ionentyps, wobei es sich vorliegend ebenfalls um Anionen 8 (wenn auch von einem unterschiedlichen Ionentyp) handelt Durch die 'wechselweise" Erzeugung von unterschiedlichen lortentypen kommt es zu einer Entladung der statisch geladenen Wandbereiche 10, sodass dank der Regenerationsphase 15 die Konzentration der in den Kraftfahrzeuginnenraum 5 freigegebenen Anionen 8 nicht nur nicht weiter abnimmt, sondern im Gegenteil wieder zunehmen kann. Nach Ablauf der Regenerationsphase 15 (welche typischerweise 5 bis 30 Sekunden andauert) schließt sich erneut ein normaler Betriebsmodus 14 an, welcher wiederum von einem Regenerationsmodus 15 gefolgt wird usw. Dieses Verfahren ist in Fig. 3 dargestellt. Auch hier ist längs der Abszisse 12 die Zeit dargestellt, wohingegen längs der Ordinate 13 die Konzentration der in den Kraftfahrzeuginnenraum 5 freigesetzten Anionen dargestellt ist.

Ein zusätzlich mögliches Verfahren zur Ansteuerung eines Ionisierungsmoduls 7 besteht darin, dass das Ionisierungsmodul 7 zunächst in eine normalen Betriebsmodus 14 betrieben wird (vergleiche mit Fig. 4). Nach Ablauf einer gewissen Zeitdauer (typischerweise 1 bis 3 Minuten) wird das Ionisierungsmodul 7 Dadurch regeneriert 16, dass das Ionisierungsmodul 7 einfach ausgeschaltet wird, und somit keinerlei Ionen (insbesondere keine Anionen 8) erzeugt werden. In dieser Regenerationsphase 16 können sich ebenfalls die statischen Aufladungen entlang der Wandbereiche 10 des Luftleitkanals 9 sowie der Luftausströmerdüse 6 abbauen, beispielsweise durch die üblicherweise im Luftstrom L befindliche Feuchtigkeit. Die Dauer der Regenerationsphase beträgt typischerweise 5 bis 30 Sekunden. Nach Ablauf der Regenerationsphase 16 erfolgt erneut ein Zyklus, in dem das Ionisierungsmodul 7 erneut in einem normalen Betriebsmodus 14 betrieben wird, anschließend erneut durch Ausschalten regeberiert 16 wird usw.. Das beschriebene Zusatz-Verfahren ist in Fig. 4 näher illustriert. Auch hier ist entlang der Abszisse 12 die Zeit aufgetragen, wohingegen längs der Ordinate 13 die in den Kraftfahrzeuginnenraum 5 freigegebene Konzentration an Anionen 8 dargestellt ist.
Obwohl mit den vorgeschlagenen Betriebsverfahren, insbesondere mit dem Betriebsverfehren gemäß Fig. 3 und ggf. Fig. 4 bereits eine im zeichen Mittel gegenüber bekannten Betriebsverfahren (vergleiche mit Fig. 2) deutlich erhöhte Ionenkonzentration in den Kraftfahrzeuginnenraum 5 freigegeben werden kann, empfiehlt es sich, zusätzlich zu den vorgeschlagenen Betriebsmodi bzw, gegebenenfalls zusätzlich zu sonstigen denkbaren vorteilhaften Betriebsmodi) zusätzliche konstruktive Maßnahmen vorzusehen, die aufgrund ihrer konstruktiven Eigenschaften den Anteil der in den Kraftfahrzeuginnenraum 5 freigegebenen Anionen 8 (oder sonstiger ioneh) nochmals erhöhen.
Eine Möglichkeit für ein derartiges konstruktives Merkmal besteht beispielesweise in der bereits in Fig, 1 angedeuteten Anordnung von einem Raster 17 von jeweils zueinander beabstandet angeordneter Metallfolien 18. Mögliche Detäils zur Lage und Anordnung der Metallfolien 18 am Luftleitkanal 9 sind darüber hinaus in Fig. 5 zu erkennen. Die Metallfolien 18 sind als selbstklebende Metallfolien ausgebildet, und werden beispielsweise auf den bereits fertig ausgebildeten Luftleitkanal 9 (der beispielsweise mit Hilfe eines Spritzgussverfahrens aus Kunststoff gefertigt wunde) aufgeklebt. Wie aus den Fig. 1 und 5 zu erkennen ist, befinden sich die Metallfolien 18 dabei auf der Außensaite 19 des Luftleitkanals 9. Im vorliegend dargestellten Ausführungsbeispiel sind die Metallfolien 18 jeweils voneinander elektrische isoliert angeordnet und darüber hinaus nicht geerdet, In einem weiteren denkbaren Ausfährungsbeispiel ist es auch möglich, das (ein Teil) der Metallfollen 18 elektrisch miteinander verbunden ist (gegebenenfalls über hochohmige elektfische Leiter) und/oder (gegebenenfalls über einen hochohmigen elektrisahen Leiter) an Erdpotential gelegt sind. Unabhängig von der konkreten Detailausführung hat sich gezeigt, dass die lokal begrenzte Beweglichkeit von Ladungsträgern in den einzelnen Metallfolien 18 bewirkt, dass Ober eine begrenzte Wegstrecke hinweg in den Metallfolien 18 Spiegelfadungen entstehen können, die zu elektrischen Ladungen bzw. Ladungsclustern an den inneren Wandbereichen 10 und/oder im inneren des Luftleitkanals 9 korrespondieren können. Die dadurch gebildeten Spiegeiladungen bewirken eine Reduktion bzw. vorteilhafte Umverteilung der vorhandenen elektrischen Felder, was schlussendlich zu einem höheren Anteil durch den Luftleitkanal 9 hindurch tretender Anionen 8 führen kann (ohne dass diese an den Wandbereichen 10 des Luftleitkanals 9 bzw des Luftausströmerelements 6 verloren gehen). Dadurch, kann der Auftritt von Anionen 8 in den Kraftfahrzeuginnenraum 5 verbessert wurden.

Ein weiteres mögliches Ausführungsbeispiel für eine Vorrichtung, die eine derartige, lokal wirkende Feldkompensation bewirkt, ist in Fig. 8 dargestellt. Hier ist ebenfalls ein Luftleitkanal 9 in einer Schematischen perspektivischen Ansicht dargestellt. Auf den Außenseiten 19 des Luftleitkanals 9 sind dabei großflächig angebfachte, sogenannte ESD-Folien 20 angebracht, Die ESD-Folien 20 sind im vorliegend dargestellten Ausführungsbeispiel als selbstklebende Folien ausgebildet, die auf den fertig ausgebildeten Luftleitkanal 9 aufgeklebt werden. Aufgrund der im Verhältnis zu Metalltollen schlechten elektrischen Leitfähgkeit - im Verhältnis zu elektrischen Isolatoren jedoch guten elektrischen Leitfähigkeit resultiert auch hier effektiv eine lokale Kompensation austretender elektrischer Felder, wobei diese Kompensation nur in einem relativ eng begrenzten Flächenbereich auftritt. Es scheint sich so zu verhalten, dass über relativ kurze räumliche Distanzen hinweg (bleispielsweise einige wenige Zentimeter) der elektrischen Widerstand der ESD-Folie 20 für die Ausbildung von Spiegelladungen nicht negativ hervortritt. Über längere räumliche Entfernungen hinweg (beispielsweise 10 Zentimeter oder mehr), scheint der elektrische -Widerstand der ESD-Folien 20 dagegen effektiv eine behinderung für die Bewegung elektrischer Spiegelladungen, und damit eine Behinderung der Kompensation der elektrischen Felder darzustellen. Eine weitere Erklärungslöglichkeit für die Eigenschaften der ESD-Fofie 20 besteht darin, dass die ESD-Folie 20 nur eine geringe Längsleitfähigkeit aufweist. Diese nur geringe Längsleitfähigkeit lässt eine lokal stark variierende Bildladung (auch über kurze Distanzen hinweg) zu, beziehungspeise macht die nur geringe Längsleitfähigkeit es möglich, dass lokal stark unterschiedliche Oberfläehenladungen abgefühlt werden können. Auch, im vorliegend dargestellten Ausführungsbeispiel von Fig. 6 ist es möglich, dass die einzelnen ESO-Folien 20 (gegebenenfalls hochohmig) elektrisch miteinander Verbunden werden und/oder (gegebenenfalls über hochohmig Verbindungsleitungen) mit Masse verbunden werde.

Weitere informationen können der Patentanmeldung DE 10 2009 038298 entnommen werden.

### Bezugszeichenliste

1, Kraftfahrzeugklimaanlage
2. Gebläse
3. Filter
4. Heizkörper
5. Kraftfahrzeuginnenraum
6. Luftausströmerdüse
7. Ionisierungsmodul
8. Anionen
9. Luftleitkanal
10. Wandbereich
11. Funktionsgraf
12. Abszisse
13. Ordinate
14. normaler Betriebsmodus
15. Regenerationsmodus
16. Regeneration
17. Raster
18. Metallfolie
19. Außenseite
20. ESD-Folie

## Patentansprüche

1. Verfahren zur Ansteuerung einer Ionisierungsvorrichtung (7) zur Ionisierung von Luft (L) zur Belüftung von Kraftfahrzeuginnenräumen (5), wobei die Ionisierungsvorrichtung (7) zeitweise in einem normalen Betriebsmodus (14) Ionen (8) eines ersten Ionentyps (8) freisetzt, wobei die Ionisierungsvorrichtung (7) zeitweise in zumindest einem Regenerationsmodus (15, 16) betrieben wird, in dem Ionen mit einem zweiten, vom ersten Ionentyp (8) unterschiedlichen Ionentyp freigesetzt werden, **dadurch gekennzeichnet, dass** die Ionisierungsvorrichtung in aufeinanderfolgenden Zyklen betrieben wird, in denen die Ionisierungsvorrichtung jeweils für einen gewissen Zeitraum zunächst im normalen Betriebsmodus (14), dann im Regenerationsmodus betrieben wird.

2. Verfahren nach Anspruch 1, **dadurch gekennzeichnet, dass** die Ionen vom zweiten Ionentyp zumindest zeitweise zusätzlich und/oder zumindest zeitweise alternativ zu den Ionen des ersten Ionentyps (8) freigesetzt werden.

3. Verfahren nach einem der vorangehenden Ansprüche, **dadurch gekennzeichnet, dass** bei einem weiteren Regenerationsmodus (16) zeitweise keine Ionen (8) freigesetzt werden.

4. Verfahren nach einem der vorangehenden Ansprüche, **dadurch gekennzeichnet, dass** zumindest zeitweise und/oder zumindest bereichsweise eine lokale Feldkompensation von elektrischen Feldern durch Spiegelladungen erfolgt, insbesondere unter Verwendung von passiv wirkenden konstruktiven Maßnahmen (18, 20).

5. Verfahren nach einem der vorangehenden Ansprüche, **dadurch gekennzeichnet, dass** der Regenerationsmodus (15, 16) länger als 2 s, 5 s, 10 s, 20 s, 30 s, 45 s und/oder 1 min und/oder kürzer als 20 s, 30 s, 45 s, 1 min, 2 min, 3 min, 4 min und/oder 5 min andauert.

6. Klimaanlage mit einer Ionisierungsvorrichtung (7) zur Ionisierung von Luft (L) zur Belüftung von Kraftfahrzeuginnenräumen (5), aufweisend Ionisierungsmittel (7) zur zeitweisen Freisetzung von Ionen (8) unterschiedlichen Ionentyps (8), **dadurch gekennzeichnet, dass** die Ionisierungsvorrichtung (7) zeitweise in einem normalen Betriebsmodus (14) Ionen (8) eines ersten Ionentyps (8) freisetzt, wobei die Ionisierungsvorrichtung (7) zeitweise in zumindest einem Regenerationsmodus (15, 16) betrieben wird, in dem Ionen mit einem zweiten, vom ersten Ionentyp (8) unterschiedlichen Ionentyp freigesetzt werden, wobei die Ionisierungsvorrichtung in aufeinanderfolgenden Zyklen betrieben wird, in denen die Ionisierungsvorrichtung jeweils für einen gewissen Zeitraum zunächst im normalen Betriebsmodus (14), dann im Regenerationsmodus betrieben wird.

7. Klimaanlage nach Anspruch 6, **dadurch gekennzeichnet, dass** die Ionen vom zweiten Ionentyp zumindest zeitweise zusätzlich und/oder zumindest zeitweise alternativ zu den Ionen des ersten Ionentyps (8) freigesetzt werden.

8. Klimaanlage nach einem der Ansprüche 6 oder 7, **dadurch gekennzeichnet, dass** bei einem weiteren Regenerationsmodus (16) zeitweise keine Ionen (8) freigesetzt werden.

9. Klimaanlage nach einem der vorangehenden Ansprüche 6 bis 8, **dadurch gekennzeichnet, dass** zumindest zeitweise und/oder zumindest bereichsweise eine lokale Feldkompensation von elektrischen Feldern durch Spiegelladungen erfolgt, insbesondere unter Verwendung von passiv wirkenden konstruktiven Maßnahmen (18, 20).

10. Klimaanlage nach einem der vorangehenden Ansprüche 6 bis 9, **dadurch gekennzeichnet, dass** der Regenerationsmodus (15, 16) länger als 2 s, 5 s, 10 s, 20 s, 30 s, 45 s und/oder 1 min und/oder kürzer als 20 s, 30 s, 45 s, 1 min, 2 min, 3 min, 4 min und/oder 5 min andauert.

11. Klimaanlage nach einem der Ansprüche 6 bis 10, **gekennzeichnet durch** eine Steuervorrichtung zur Ansteuerung der Ionisierungsvorrichtung (7), insbesondere zur Durchführung des Regenerationsmodus (18, 20).

12. Klimaanlage nach einem der Ansprüche 6 bis 11, **gekennzeichnet durch** ein erstes Ionisierungsmittel (7) zur zumindest zeitweisen Freisetzung des ersten Ionentyps und zumindest ein zweites Ionisierungsmittel, insbesondere zur zumindest zeitweisen Freisetzung von Ionen des zweiten Ionentyps.

13. Klimaanlage nach einem der Ansprüche 6 bis 12, **gekennzeichnet durch** zumindest eine Luftführungsvorrichtung (6, 9), insbesondere einen Luftführungskanal (9) und/oder eine Luftauslassdüse (6), welcher zumindest bereichsweise ein lokal wirkendes Feldkompensationsmittel (18, 20) aufweist.

14. Klimaanlage nach Anspruch 13, **dadurch gekennzeichnet, dass** zumindest ein lokal wirkendes Feldkompensationsmittel (18, 20) zumindest bereichsweise als eine Mehrzahl von elektrisch leitfähigen Materialabschnitten (18) ausgebildet ist.

15. Klimaanlage nach Anspruch 13 oder 14, **dadurch gekennzeichnet, dass** zumindest ein lokal wirkendes Feldkompensationsmittel (18, 20) zumindest bereichsweise als durchgängiges, elektrisch geringfügig leitendes Bauteil (20) ausgebildet ist.

16. Klimaanlage nach einem der Ansprüche 13 bis 15, **dadurch gekennzeichnet, dass** zumindest ein lokal wirkendes Feldkompensationsmittel (18, 20), insbesondere zumindest ein elektrisch leitfähiger Materialabschnitt (18) und/oder zumindest ein elektrisch geringfügig leitendes Bauteil (20) flächig, bevorzugt folienartig ausgebildet ist.

17. Klimaanlage nach einem der Ansprüche 13 bis 16, **dadurch gekennzeichnet, dass** zumindest ein lokal wirkendes Feldkompensationsmittel (18, 20), insbesondere zumindest ein elektrisch leitfähiger Materialabschnitt (18) und/oder zumindest ein elektrisch geringfügig leitendes Bauteil (20) selbstklebend ausgeführt ist.

## Claims

1. A method for controlling an ionisation device (7) for ionising air (L) for ventilating interior spaces of vehicles (5), wherein the ionisation device (7) intermittently releases ions (8) of a first ion type (8) in a normal operating mode (14), wherein the ionisation device (7) is operated intermittently in at least one regeneration mode (15, 16) in which ions with a second ion type, different from the first ion type (8), are released, **characterised in that** the ionisation device is operated in consecutive cycles in which the ionisation device is in each case operated first in the normal operation mode (14), then in the regeneration mode for a certain period of time.

2. The method according to claim 1, **characterised in that** the ions of the second ion type are released at least intermittently in addition and/or at least intermittently alternatively to the ions of the first ion type (8).

3. The method according to one of the preceding claims, **characterised in that** in a further regeneration mode (16) intermittently no ions (8) are released.

4. The method according to one of the preceding claims, **characterised in that** at least intermittently and/or at least in areas a local field compensation of electric fields by image charges occurs, especially during use of passively acting design measures (18, 20) .

5. The method according to one of the preceding claims, **characterised in that** the regeneration mode (15, 16) lasts longer than 2 s, 5 s, 10 s, 20 s, 30 s, 45 s and/or 1 min and/or less than 20 s, 30 s, 45 s, 1 min, 2 min, 3 min, 4 min and/or 5 min.

6. An air-conditioning system with an ionisation device (7) for ionising air (L) for ventilating interior spaces of vehicles (5), having ionising means (7) for the intermittent release of ions (8) of different ion types (8), **characterised in that** the ionisation device (7) intermittently releases ions (8) of a first ion type (8) in a normal operating mode (14), wherein the ionisation device (7) is operated intermittently in at least one regeneration mode (15, 16) in which ions with a second ion type, different from the first ion type (8), are released, wherein the ionisation device is operated in consecutive cycles in which the ionisation device is in each case operated first in the normal operation mode (14), then in the regeneration mode for a certain period of time.

7. The air-conditioning system according to claim 6, **characterised in that** the ions of the second ion type are released at least intermittently in addition and/or at least intermittently alternatively to the ions of the first ion type (8).

8. The air-conditioning system according to one of claims 6 or 7, **characterised in that** in a further regeneration mode (16) intermittently no ions (8) are released.

9. The air-conditioning system according to one of the preceding claims 6 to 8, **characterised in that** at least intermittently and/or at least in areas a local field compensation of electric fields by image charges occurs, especially during use of passively acting design measures (18, 20).

10. The air-conditioning system according to one of the preceding claims 6 to 9, **characterised in that** the regeneration mode (15, 16) lasts longer than 2 s, 5 s, 10 s, 20 s, 30 s, 45 s and/or 1 min and/or less than 20 s, 30 s, 45 s, 1 min, 2 min, 3 min, 4 min and/or 5 min.

11. The air-conditioning system according to one of claims 6 to 10, **characterised by** a control device for controlling the ionisation device (7), particularly for carrying out the regeneration mode (18, 20).

12. The air-conditioning system according to one of claims 6 to 11, **characterised by** a first ionising means (7) for the at least intermittent release of the first ion type and at least a second ionising means, particularly for the at least intermittent release of ions of the second ion type.

13. The air-conditioning system according to one of claims 6 to 12, **characterised by** at least one air guiding device (6, 9), particularly a ventilation duct (9) and/or an air outlet nozzle (6), which has a locally acting field compensation means (18, 20) at least in areas.

14. The air-conditioning system according to claim 13, **characterised in that** at least one locally acting field compensation means (18, 20) is formed at least in areas as a plurality of electrically conductive material sections (18).

15. The air-conditioning system according to claim 13 or 14, **characterised in that** at least one locally acting field compensation means (18, 20) is formed at least in areas as a continuous, electrically slightly conductive component (20).

16. The air-conditioning system according to one of claims 13 to 15, **characterised in that** at least one locally acting field compensation means (18, 20), particularly at least one electrically conductive material section (18) and/or at least one electrically slightly conductive component (20) is formed flat, preferably film-like.

17. The air-conditioning system according to one of claims 13 to 16, **characterised in that** at least one locally acting field compensation means (18, 20), particularly at least one electrically conductive material section (18) and/or at least one electrically slightly conductive component (20) are made to be self-adhesive.

## Revendications

1. Procédé conçu pour l'activation d'un dispositif d'ionisation (7) destiné à l'ionisation de l'air (L) utilisé pour la ventilation d'habitacles (5) de véhicules automobiles, où le dispositif d'ionisation (7), en mode de fonctionnement normal (14), libère temporairement des ions (8) d'un premier type d'ions (8), où le dispositif d'ionisation (7) est actionné temporairement dans au moins un mode de régénération (15, 16) au cours duquel sont libérés des ions d'un deuxième type différent du premier type d'ions (8), **caractérisé en ce que** le dispositif d'ionisation est actionné suivant des cycles successifs au cours desquels le dispositif d'ionisation est actionné à chaque fois pendant un certain laps de temps, d'abord en mode de fonctionnement normal (14) puis en mode de régénération.

2. Procédé selon la revendication 1, **caractérisé en ce que** les ions du deuxième type d'ions sont libérés au moins temporairement en plus et / ou au moins temporairement en alternance avec les ions du premier type d'ions (8).

3. Procédé selon l'une des revendications précédentes, **caractérisé en ce qu'**aucun ion (8) n'est libéré temporairement au cours d'un autre mode de régénération (16).

4. Procédé selon l'une quelconque des revendications précédentes, **caractérisé en ce qu'**une compensation de champ effectuée localement, concernant des champs électriques créés par des charges spéculaires, se produit au moins temporairement et / ou au moins partiellement, en particulier en utilisant des mesures structurelles (18, 20) agissant passivement.

5. Procédé selon l'une quelconque des revendications précédentes, **caractérisé en ce que** le mode de régénération (15, 16) dure plus longtemps que 2 s, 5 s, 10 s, 20 s, 30 s, 45 s et / ou 1 min et / ou dure moins longtemps que 20 s, 30 s, 45 s, 1 min, 2 min, 3 min, 4 min et / ou 5 min.

6. Système de climatisation comprenant un dispositif d'ionisation (7) destiné à l'ionisation de l'air (L) utilisé pour la ventilation d'habitacles (5) de véhicules automobiles, ledit dispositif d'ionisation présentant des moyens d'ionisation (7) prévus pour la libération temporaire d'ions (8) de types d'ions différents (8), **caractérisé en ce que** le dispositif d'ionisation (7), en mode de fonctionnement normal (14), libère temporairement des ions (8) d'un premier type d'ions (8), où le dispositif d'ionisation (7) est actionné temporairement dans au moins un mode de régénération (15, 16) au cours duquel sont libérés des ions d'un deuxième type différent du premier type d'ions (8), où le dispositif d'ionisation est actionné suivant des cycles successifs au cours desquels le dispositif d'ionisation est actionné à chaque fois pendant un certain laps de temps, d'abord en mode de fonctionnement normal (14) puis en mode de régénération.

7. Système de climatisation selon la revendication 6, **caractérisé en ce que** les ions du deuxième type d'ions sont libérés au moins temporairement en plus et / ou au moins temporairement en alternance avec les ions du premier type d'ions (8).

8. Système de climatisation selon l'une des revendications 6 ou 7, **caractérisé en ce qu'**aucun ion (8) n'est libéré temporairement au cours d'un autre mode de régénération (16).

9. Système de climatisation selon l'une quelconque des revendications précédentes 6 à 8, **caractérisé en ce qu'**une compensation de champ effectuée localement, concernant des champs électriques créés par des charges spéculaires, se produit au moins temporairement et / ou au moins partiellement, en particulier en utilisant des mesures structurelles (18, 20) agissant passivement.

10. Système de climatisation selon l'une quelconque des revendications précédentes 6 à 9, **caractérisé en ce que** le mode de régénération (15, 16) dure plus longtemps que 2 s, 5 s, 10 s, 20 s, 30 s, 45 s et / ou 1 min et / ou dure moins longtemps que 20 s, 30 s, 45 s, 1 min, 2 min, 3 min, 4 min et / ou 5 min.

11. Système de climatisation selon l'une quelconque des revendications 6 à 10, **caractérisé par** un dispositif de commande pour l'activation du dispositif d'ionisation (7), servant en particulier à la mise en oeuvre du mode de régénération (18, 20).

12. Système de climatisation selon l'une quelconque des revendications 6 à 11, **caractérisé par** un premier moyen d'ionisation (7) servant à la libération au moins temporaire d'ions du premier type d'ions, et **caractérisé par** au moins un deuxième moyen d'ionisation servant en particulier à la libération au moins temporaire d'ions du deuxième type d'ions.

13. Système de climatisation selon l'une quelconque des revendications 6 à 12, **caractérisé par** au moins un dispositif de guidage d'air (6, 9), en particulier un conduit de guidage d'air (9) et / ou une buse de sortie d'air (6), conduit de guidage d'air qui présente au moins partiellement un moyen de compensation de champ (18, 20) agissant localement.

14. Système de climatisation selon la revendication 13, **caractérisé en ce qu'**au moins un moyen de compensation de champ (18, 20) agissant localement est configuré au moins partiellement comme une pluralité de parties de matière (18) électroconductrices.

15. Système de climatisation selon la revendication 13 ou 14, **caractérisé en ce qu'**au moins un moyen de compensation de champ (18, 20) agissant localement est configuré au moins partiellement comme un composant électrique continu (20) faiblement conducteur.

16. Système de climatisation selon l'une quelconque des revendications 13 à 15, **caractérisé en ce qu'**au moins un moyen de compensation de champ (18, 20) agissant localement, en particulier au moins une partie de matière (18) électroconductrice et / ou au moins un composant électrique (20) faiblement conducteur, est configuré de façon plate, de préférence en forme de feuille.

17. Système de climatisation selon l'une quelconque des revendications 13 à 16, **caractérisé en ce qu'**au moins un moyen de compensation de champ (18, 20) agissant localement, en particulier au moins une partie de matière (18) électroconductrice et / ou au moins un composant électrique (20) faiblement conducteur, est réalisé en étant autoadhésif.
